# EUROPÄISCHE PATENTSCHRIFT

(11) **EP 1 862 213 B1**
(45) Veröffentlichungstag und Bekanntmachung des Hinweises auf die Patenterteilung: **18.11.2009**
(21) Anmeldenummer: 07009747.2
(22) Anmeldetag: 16.05.2007
(51) Int. Cl.: B01D 61/10, B01D 65/02, C02F 1/44, B01D 61/12, A61L 2/18

(54) **Vorrichtung zur Desinfektion einer Umkehrosmoseanlage**
Device for disinfecting a reverse osmosis facility
Dispositif destiné à la désinfection d'une installation d'osmose inverse

(30) Priorität: 03.06.2006 DE 102006026107
(43) Veröffentlichungstag der Anmeldung: 05.12.2007
(73) Patentinhaber: Völker, Manfred, 63825 Blankenbach (DE)
(72) Erfinder: Völker, Manfred, 63825 Blankenbach (DE)
(74) Vertreter: Flosdorff, Jürgen

(56) Entgegenhaltungen:
- EP-A- 1 820 518
- WO-A-99/55448
- DE-U1- 29 901 168
- FR-A1- 2 428 240
- GB-A- 2 010 779
- US-A- 4 158 034

## Beschreibung

Die Erfindung betrifft eine Umkehrosmoseanlage gemäß dem Oberbegriff des Patentanspruchs 1.

Umkehrosmoseanlagen dienen insbesondere zur Gewinnung reinen, keimfreien Wassers aus Leitungswasser, z.B. für medizinische und lebensmitteltechnische Anwendungen. Ihr Funktionsprinzip besteht bekanntlich darin, daß das aufzubereitende Wasser in einem Filtermodul unter hohem Druck an der Oberfläche einer semipermeablen Membran entlanggeführt wird, wobei ein Teil des Wassers, das sogenannte Permeat, durch die Membran tritt und auf der anderen Seite der Membran gesammelt und der Verbrauchsstelle zugeführt wird. Der nicht durch die Membran tretende, mit zurückgehaltenen Stoffen angereicherte Teil des Rohwassers, das sogenannte Konzentrat, fließt am Ende der Strömungsstrecke des Primärraumes aus dem Membranmodul aus.

Das so gewonnene Reinstwasser ist im Idealfalle aufgrund der Rückhalteeigenschaften der Membran keimfrei und frei von organischen Zerfallsprodukten. In der Realität trifft dies jedoch nicht ohne weiteres zu. Ohne besondere Gegenmaßnahmen kann es zu einer Besiedlung des Permeatsystems mit Mikroorganismen kommen. Es bildet sich ein sogenannter Biofilm auf den Innenflächen des flüssigkeitsführenden Systems.

Daher ist es notwendig, an Umkehrosmoseanlagen in geeigneten Zeitabständen eine Desinfektion vorzunehmen. Hierzu wird der normale Betrieb unterbrochen und dem flüssigkeitsführenden System ein chemisches Desinfektionsmittel zugeführt. Nach einer geeigneten Einwirkungszeit folgt ein Spülvorgang, der dazu dient, das eingebrachte Desinfektionsmittel und seine Reaktionsprodukte wieder zu entfernen, so daß anschließend der normale Versorgungsbetrieb wieder aufgenommen werden kann.

Wegen der erheblichen Gefahren, die mit einer unkontrollierten Zufuhr eines Desinfektionsmittels verbunden sind, insbesondere bei Anwendungen im medizinischen Bereich (Hämodialyse), müssen alle für die Durchführung der Desinfektion notwendigen Arbeitsschritte üblicherweise manuell, unter Kontrolle des Bedienungspersonals, durchgeführt werden. Hierzu zählt u.a. das Herstellen und spätere Trennen der Verbindung zwischen der Konnektionsstelle der Umkehrosmoseanlage und einem externen Desinfektionsmittelbehälter und die Überwachung der korrekten Zufuhr des Desinfektionsmittels. Dies bedeutet im allgemeinen einen erheblichen Arbeitsaufwand.

Die WO 99/55448 A offenbart ein automatisches Reinigungssystem für eine Umkehrosmoseeinheit, bei der ein chemisches Injektionssystem in Fluidverbindung mit dem Leitungssystem der Umkehrosmoseeinheit steht. Es sind keine Vorkehrungen getroffen, um mit Sicherheit auszuschließen, dass durch eine irrtümliche Betätigung des chemischen Injektionssystems während des Normalbetriebs der Umkehrosmoseeinheit das chemische Reinigungsmittel in den Kreislauf gerät.

Die FR 2 428 240 A1 offenbart eine Vorrichtung zur Dosierung eines Zusatzmittels für Waschmaschinen, bei der eine vorbestimmte Dosiermenge durch eine Pumpe in eine Ansaugkammer angesaugt wird. Diese Dosiermenge wird dann durch die Pumpe in die Waschmaschine abgegeben.

Die Erfindung zielt darauf ab, die sicherheitstechnischen Voraussetzungen zu verbessern und dadurch eine weitgehend automatisierte Durchführung der Desinfektion einer Umkehrosmoseanlage zu ermöglichen.

Diese Aufgabe wird erfindungsgemäß durch die Merkmale des Patentanspruchs 1 gelöst. Vorteilhafte Ausgestaltungen der Erfindung sind in den Unteransprüchen gekennzeichnet.

Weitere Einzelheiten und Vorteile der Erfindung ergeben sich aus der nachfolgenden Beschreibung von Ausführungsbeispielen in Verbindung mit den Abbildungen. Es zeigen
- Figur 1: das Schema einer Unkehrosmoseanlage mit Ausstattungsmerkmalen entsprechend der Erfindung;
- Figur 2: eine Vorrichtung gemäß der Erfindung zur Zuführung des Desinfektionsmittels;
- Figur 3: eine andere Version der Vorrichtung zur Zuführung des Desinfektionsmittels.

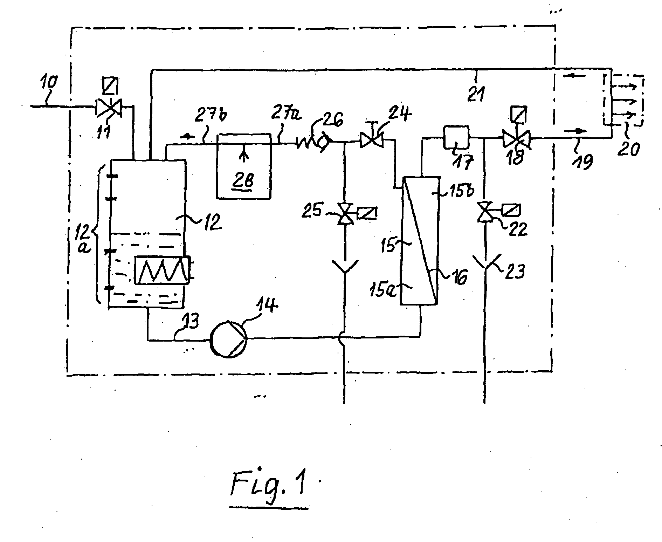

Die prinzipielle Einrichtung einer Umkehrosmoseanlage ist schematisch in Figur 1 dargestellt. Das über die Leitung 10 zugeführte Rohwasser gelangt über das Einlaßventil 11 in den Vorlaufbehälter 12. Aus dem Vorlaufbehälter wird über die Leitung 13 mittels der Pumpe 14 das Umkehrosmose-Filtermodul (RO-Modul) 15 gespeist, dessen Primärraum 15a durch eine semipermeable Membran 16 von dem Sekundärraum 15b getrennt ist. Das Permeat fließt aus dem Sekundärraum über eine Analysevorrichtung 17 - z.B. in Form eines elektrischen Leitfähigkeitsmessers - und ein Permeat-Speiseventil 18 in die zu den Verbrauchsstellen 20 führende Leitung 19. Überschüssiges Permeat, das an den Verbrauchsstellen nicht abgegeben wird, fließt über die Rückführungsleitung 21 zum Eingang der Anlage zurück und vermischt sich im Vorlaufbehälter 12 mit dem zugeführten Rohwasser.

Von der zum Permeat-Speiseventil 18 führenden Leitung zweigt eine Leitung mit dem Ventil 22 zum Abfluß 23 ab. Durch Öffnen des Ventils 22 und Schließen des Ventils 18 kann somit das erzeugte Permeat verworfen werden. Dies dient insbesondere dazu, bei einer durch die Analysevorrichtung 17 detektierten unzureichenden Qualität die Weiterleitung des Permeats zu den Verbrauchsstellen automatisch abzubrechen.

Ein am Ausgang des Primärraums des Filtermoduls eingefügter Strömungswiderstand, z.B. in Form einer Drossel 24, ist maßgebend für den im Primärraum entstehenden Druck, der für die Filtration notwendig ist. Das aus dem Primärraum abfließende Konzentrat wird bei geöffnetem Ventil 25 in den Abfluß geleitet. Bei geschlossenem Ventil 25 fließt das Konzentrat über das Rückschlagventil 26 und die Leitung 27a/b in den Vorlaufbehälter 12 zurück. Ein zeitweiliges Schließen des Ventils 25 während des Versorgungsbetriebes kommt insbesondere in Betracht, wenn die Permeatentnahme an den Verbrauchsstellen gering ist und der Rohwasserverbrauch entsprechend reduziert werden soll (Ausbeuteregelung).

Bei der in Figur 1 dargestellten Anordnung ist die erfindungsgemäß vorgesehene Vorrichtung 28 zur Zufuhr eines Desinfektionsmittels an die Konzentrat-Rückführungsleitung angeschlossen bzw. zwischen den Leitungsabschnitten 27a und 27b eingefügt.

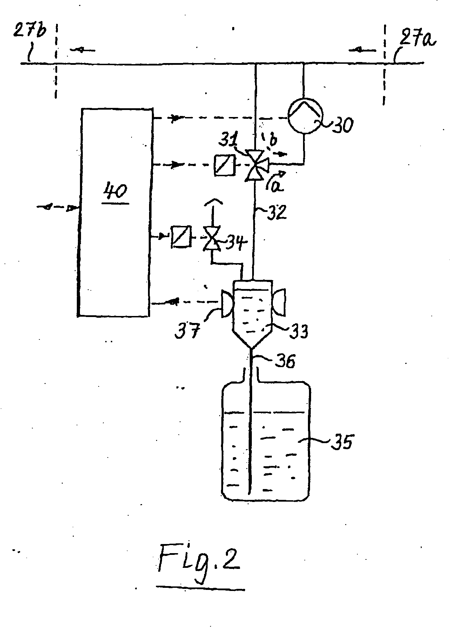

Einzelheiten eines Ausführungsbeispiels dieser Vorrichtung 28 sind in Figur 2 dargestellt. Wesentliche Bestandteile sind eine Pumpe 30, deren Sauganschluß über das Pumpenventil 31 und die Leitung 32 mit dem oberen Teil einer Ansaugkammer 33 verbunden ist, und eine ebenfalls mit dem oberen Teil der Ansaugkammer 33 verbundene Belüftungsleitung mit dem Belüftungsventil 34. Das Desinfektionsmittel wird in einem belüfteten Vorratsbehälter 35 bereitgehalten, dessen Füllpegel unterhalb der Ansaugkammer liegt und mit dem unteren Teil der Ansaugkammer über eine Saugleitung 36 verbunden ist. An der Ansaugkammer 33 befindet sich ein Füllstandssensor 37, der die Information darüber liefert, ob der Flüssigkeitspegel in der Ansaugkammer ein bestimmtes Niveau unter- oder überschreitet.

Zur Zuführung des Desinfektionsmittels wird das Belüftungsventil 34 geschlossen. An dem Pumpenventil 31 wird der Strömungsweg "a", der die Verbindung zur Ansaugkammer herstellt, geöffnet und gleichzeitig der Strömungsweg "b", der Saug- und Druckseite der Pumpe 30 überbrückt, geschlossen. Durch Einschalten der Pumpe 30 wird die Luft aus der Ansaugkammer abgesaugt, und durch den entstehenden Unterdruck strömt Desinfektionsmittel aus dem Vorratsbehälter 35 nach, so daß schließlich über die Pumpe das Desinfektionsmittel in die Leitung 27a/b gefördert wird.

Die Zuführung wird fortgesetzt, bis eine vorgegebene Volumenzunahme im Vorlaufbehälter 12 erreicht ist, die mittels Füllstandssensoren 12a nachgewiesen werden kann, oder bis ein vorgegebener Konzentrationsanstieg stattgefunden hat. Letzteres kann z.B. aufgrund eines entsprechenden Anstieges der elektrischen Leitfähigkeit festgestellt werden, die an geeigneter Stelle im Permeat-Verteilungssystem gemessen wird.

Dem Füllstandssensor 37 an der Ansaugkammer 33 kommt bei diesem Vorgang eine wichtige Überwachungsfunktion zu: Bei Erschöpfung des Desinfektionsmittel-Vorrats im Behälter 35 wird der durch die Position des Sensors vorgegebene Füllstand in der Ansaugkammer nicht erreicht, oder es kommt während der Desinfektionsmittelzufuhr zu einem Absinken des Füllstandes unter diese Position, was jeweils zur Auslösung eines entsprechenden Alarmsignals genutzt werden kann.

Von besonderer Bedeutung ist die Sicherheitsfunktion der gezeigten Anordnung im normalen Versorgungsbetrieb der Umkehrosmoseanlage, da sie geeignet ist, eine unbeabsichtigte Desinfektionsmittelszufuhr aus dem angeschlossenen Vorratsbehälter 35 zu verhindern. Die Ansaugstrecke wird durch das geöffnete Ventil 34 permanent belüftet, so daß das Desinfektionsmittel nicht in die Ansaugkammer 33 aufsteigen kann, auch nicht im Falle einer Fehlfunktion anderer Komponenten (Pumpe 30, Ventil 31). Darüber hinaus würde ein unzulässiges Aufsteigen von Desinfektionsmittel in die Ansaugkammer 33 durch den Füllstandssensor 37 erkannt, was zur Auslösung eines entsprechenden Alarmsignals und im Bedarfsfalle zu einer Sicherheitsabschaltung der Anlage genutzt werden kann.

Im Anschluß an eine chemische Desinfektion ist nach Zuführung und Verteilung des Desinfektionsmittels und nach Ablauf einer vorbestimmten Einwirkungszeit eine Spülung notwendig, um die Restkonzentration des Desinfektionsmittels zu minimieren. Die Anordnung gemäß Figur 2 bietet die Möglichkeit, die Pumpe 30 in die Spülung einzubeziehen, indem sie zeitweilig eingeschaltet und dabei der Strömungsweg "b" des Ventils 31 geöffnet wird.

Die beschriebenen Steuerungs- und Überwachungsfunktionen werden nach Bedarf unter Vermittlung einer entsprechen eingerichteten Steuerungseinheit 40 realisiert, die auch die funktionelle Verbindung mit anderen Funktionselementen der Umkehrosmose herstellt.

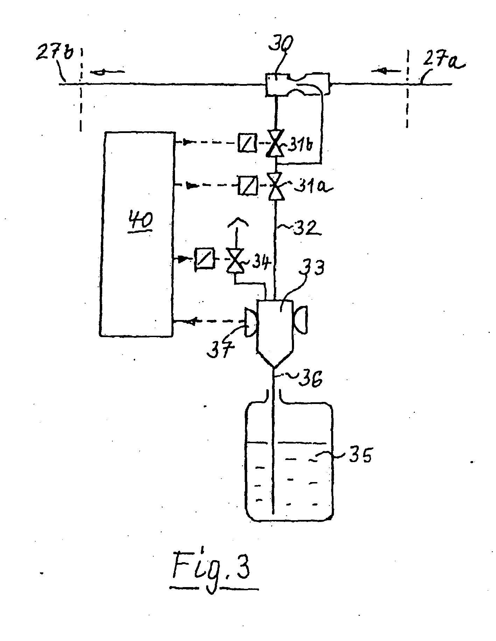

Bei dem Ausführungsbeispiel gemäß Figur 3 ist die Pumpe 30 als Wasserstrahlpumpe ausgebildet, die sich vorteilhaft durch Einfachheit und Robustheit auszeichnet. Voraussetzung hierfür ist, daß in der Leitung 27a/b ein relativ niedriger Druck herrscht. Bei der Anordnung gemäß Figur 1 ist dies der Fall, da der Leitungsabschnitt 27b in den atmosphärisch belüfteten Vorlaufbehälter 12 mündet. Bei Einsatz einer extern angetriebenen Pumpe gemäß Figur 2 entfällt diese Voraussetzung, d.h. sie kann auch an anderer geeigneter Stelle in das Leitungssystem der Umkehrosmoseanlage eingefügt werden.

Das 3-Wege-Ventil 31 in Figur 2 ist bei dem Ausführungsbeispiel gemäß Figur 3 durch zwei Einzelventile 31 a und 31 b ersetzt. Die Funktion ist jedoch prinzipiell gleichartig.

Es wird betont, dass die Erfindung nicht auf die beschriebenen und dargestellten Ausführungsformen beschränkt ist. Vielmehr sind alle offenbarten Merkmale der Vorrichtung auf jede Weise einzeln miteinander kombinierbar.

## Patentansprüche

1. Umkehrosmoseanlage mit einem Umkehrosmose-Filtermodul (15), einem Leitungssystem mit einer Rohwasser-Zuführleitung (10), einer Permeatleitung (19, 21) und einer Konzentrat-Rückführleitung (27a, 27b), und mit einer Pumpe (30), die dazu geeignet ist, über eine Saugleitung (32) aus einem atmosphärisch belüfteten Vorratsbehälter (35) entnommenes chemisches Desinfektionsmittel in das Leitungssystem der Umkehrosmoseanlage zu fördern,
**gekennzeichnet durch** eine in die Saugleitung eingefügte Ansaugkammer (33) mit einem oberen Anschluß zu der Pumpe (30) und einem unteren Anschluß zu dem Vorratsbehälter (35) sowie mit einer mit dem oberen Teil verbundenen Belüftungsleitung, die mit einem Absperrorgan (34) versehen ist, das im Betriebszustand der Desinfektionsmittelzufuhr geschlossen und während der übrigen Betriebszustände der Umkehrosmoseanlage geöffnet ist.

2. Umkehrosmoseanlage nach Anspruch 1,
**dadurch gekennzeichnet,**
**daß** die Ansaugkammer (33) mit einem Füllstandssensor (37) ausgestattet ist.

3. Umkehrosmoseanlage nach Anspruch 2,
**dadurch gekennzeichnet,**
**daß** der Füllstandssensor (37) mit einer Steuerungsanlage (40) verbunden ist, die ein Alarmsignal auslöst und/oder die Umkehrosmoseanlage ausschaltet, wenn der Füllstandssensor (37) ein Aufsteigen von Desinfektionsmittel in die Ansaugkammer (33) während der übrigen Betriebszustände erfaßt.

4. Umkehrosmoseanlage nach Anspruch 3,
**dadurch gekennzeichnet,**
**daß** die Steuerungsanlage (40) ferner ein Alarmsignal auslöst, wenn der Füllstandssensor (37) während des Betriebszustands der Desinfektionsmittelzufuhr erfaßt, daß der Füllstand des Desinfektionsmittels die Position des Füllstandssensors (37) nicht erreicht oder unter diese absinkt.

5. Umkehrosmoseanlage nach einem der Ansprüche 3 bis 4,
**dadurch gekennzeichnet,**
**daß** zwischen der Pumpe (30) und der Ansaugkammer (33) wenigstens ein Ventil (31) angeordnet ist und daß die Pumpe (30) und das wenigstens eine Ventil (31) mit der Steuerungsanlage (40) verbunden sind.

6. Umkehrosmoseanlage nach Anspruch 5,
**dadurch gekennzeichnet,**
**daß** die Pumpe (30) eine Wasserstrahlpumpe ist, die in die Konzentrat-Rückführungsleitung (27a, 27b) eingeschaltet ist.

7. Umkehrosmoseanlage nach Anspruch 1,
**dadurch gekennzeichnet,**
**daß** das Absperrorgan (34) ein Ventil ist.

## Claims

1. A reverse osmosis installation with a reverse osmosis filter module (15), a conduit system with an untreated water supply conduit (10), a permeate conduit (19, 21) and a concentrate return conduit (27a, 27b) and with a pump (30), which is suitable for conveying chemical disinfectant from an atmospherically ventilated supply container (35) via a suction conduit (32) into the conduit system of the reverse osmosis installation, **characterised by** an inlet chamber (33), inserted into the suction conduit, with an upper connection to the pump (30) and a lower connection to the supply container (35) and with a ventilation conduit connected to the upper portion, which is provided with a shut-off element (34) which is closed in the operational state of the disinfectant supply and is open during the other operational states of the reverse osmosis installation.

2. A reverse osmosis installation as claimed in Claim 1, **characterised in that** the inlet chamber (33) is equipped with a filling level sensor (37).

3. A reverse osmosis installation as claimed in Claim 2, **characterised in that** the filling level sensor (37) is connected to a control system (40), which initiates an alarm signal and/or switches off the reverse osmosis installation when the filling level sensor (37) detects an increase of disinfectant in the inlet chamber (33) during the other operational states.

4. A reverse osmosis installation as claimed in Claim.3, **characterised in that** the control system (40) also initiates an alarm signal when the filling level sensor (37) detects, during the operational state of the disinfectant supply, that the filling level of the disinfectant has not reached the position of the filling level sensor (37) or sinks below it.

5. A reverse osmosis installation as claimed in one of Claims 3 to 4, **characterised in that** arranged between the pump (30) and the inlet chamber (33) there is at least one valve (31) and that the pump (30) and the at least one valve (31) are connected to the control system (40).

6. A reverse osmosis installation as claimed in Claim 5, **characterised in that** the pump (30) is a water jet pump, which is connected into the concentrate return conduit (27a, 27b).

7. A reverse osmosis installation as claimed in Claim 1, **characterised in that** the shut-off element (34) is a valve.

## Revendications

1. Installation d'osmose inverse, comportant un module de filtrage (15), un système de conduites avec une conduite d'admission d'eau non traitée (10), une conduite du perméat (19, 21) et une conduite de retour du concentrat (27a, 27b), et comportant une pompe (30) qui est apte à refouler, via une conduite d'aspiration (32), le désinfectant chimique, prélevé dans un réservoir de stockage (35) ventilé à l'air ambiant, vers le système de conduites de l'installation d'osmose inverse,
**caractérisée par** une chambre d'aspiration (33), qui est insérée dans la conduite d'aspiration et qui est munie d'une raccord supérieur vers la pompe (30) et d'un raccord inférieur vers le réservoir de stockage (35), et est munie d'une conduite d'aération reliée à la partie supérieure et munie d'un organe de fermeture (34), qui est fermé pendant l'admission du désinfectant et qui est ouverte pendant les autres états de fonctionnement de l'installation d'osmose inverse.

2. Installation d'osmose inverse selon la revendication 1, **caractérisée en ce que** la chambre d'aspiration (33) est équipée d'un capteur du niveau de remplissage (37).

3. Installation d'osmose inverse selon la revendication 2, **caractérisée en ce que** le capteur du niveau de remplissage (37) est relié à un dispositif de commande (40), qui délivre un signal d'alarme et/ou désactive l'installation d'osmose inverse lorsque le capteur du niveau de remplissage (37) détecte une augmentation du désinfectant dans la chambre d'aspiration (33) pendant les autres états de fonctionnement.

4. Installation d'osmose inverse selon la revendication 3, **caractérisée en ce que** le dispositif de commande (40) délivre également un signal d'alarme lorsque le capteur du niveau de remplissage (37) détecte, pendant l'admission du désinfectant, que le niveau de remplissage du désinfectant n'a pas atteint la position du capteur du niveau de remplissage (37) ou a chuté en dessous de celui-ci.

5. Installation d'osmose inverse selon l'une quelconque des revendications 3 à 4, **caractérisée en ce qu'**au moins une vanne (31) est disposée entre la pompe (30) et la chambre d'aspiration (33) et **en ce que** la pompe (30) et ladite au moins une vanne (31) sont reliées au dispositif de commande (40).

6. Installation d'osmose inverse selon la revendication 5, **caractérisée en ce que** la pompe (30) est une pompe à jet d'eau, qui est montée dans une conduite de retour du concentrat (27a, 27b).

7. Installation d'osmose inverse selon la revendication 1, **caractérisée en ce que** l'organe de fermeture (34) est une vanne.
